# EUROPEAN PATENT SPECIFICATION

(11) **EP 3 162 284 B1**
(45) Date of publication and mention of the grant of the patent: **04.12.2019**
(21) Application number: 16193547.3
(22) Date of filing: 12.10.2016
(51) Int. Cl.: A61B 5/00, A61B 5/024, G08B 21/24, A61B 5/11, G08B 13/14, G08B 21/04, H04B 1/3827, H04W 4/02

(54) **COMMUNICATION METHOD AND APPARATUS FOR WEARABLE DEVICE**
KOMMUNIKATIONSVERFAHREN UND VORRICHTUNG FÜR EINE TRAGBARE VORRICHTUNG
PROCÉDÉ ET APPAREIL DE COMMUNICATION POUR DISPOSITIF VESTIMENTAIRE

(30) Priority: 29.10.2015 CN 201510719720
(43) Date of publication of application: 03.05.2017
(73) Proprietor: Xiaomi Inc., Beijing 100085 (CN)
(72) Inventor: XING, Xinyan, Haidian District Beijing 100085 (CN); TANG, Yao, Haidian District Beijing 100085 (CN); LIU, Daokuan, Haidian District Beijing 100085 (CN); LIU, Shuai, Haidian District Beijing 100085 (CN); YANG, Chunhe, Haidian District Beijing 100085 (CN)
(74) Representative: Loustalan, Paul William

(56) References cited:
- WO-A1-2007/116211
- US-A- 6 072 396
- US-A1- 2010 285 771
- US-A1- 2013 135 097
- US-A1- 2015 262 458

## Description

### FIELD

The present disclosure generally relates to electronic technology, and more particularly to a communication method, apparatus and system for wearable device.

### BACKGROUND

With the improvement of the level of technology, various wearable devices for monitoring a user's healthy condition and exercise habit have emerged, such as intelligent bracelets and intelligent watches, etc.

In related art, a wearable device may send the acquired data on health (e.g., heart rate, sleep time and body temperature, etc.) and data on exercise (e.g., number of steps and calorie consumption, etc.) of a user to a specified terminal with which it has been associated. The terminal may store and display the received data on health and exercise.

US 6072396A describes apparatus and method for continuous electronic monitoring and tracking of individuals for safety purposes. In particular, a wristband having a motion sensor may be used to periodically detect motion, and if the wristband remains motionless for an abnormally long period of time, the monitored subject may be deemed to have lost consciousness and be in need of medical attention, or have removed the wristband.

US 2010/0285771 describes a wearable transceiver that detects a fall of the device or a crash of the wearer thereof and is configured to transmit a warning signal in case of a crash.

### SUMMARY

In view of the related arts, a communication method, apparatus and system for a wearable device are provided in the disclosure.

According to a first aspect of the present disclosure, a communication method for a wearable device is provided, including: acquiring a motion state parameter of a wearable device; detecting, at a current time, whether a temperature of a location where the wearable device contacts with a user's skin is higher than a temperature of another location of the wearable device; and, if the temperature of the location where the wearable device contacts with the user's skin is not higher than the temperature of the other location of the wearable device, determining whether the wearable device is in a fallen state, in which the wearable device is no longer being worn because it has fallen, the determination being based on the motion state parameter within a predefined time period before the current time; and if the wearable device is in the fallen state, sending information about falling to a terminal to instruct the terminal to send out an alerting signal, wherein the motion state parameter comprises speed and acceleration, and the step of determining whether the wearable device is in the fallen state based on the motion state parameter comprises:
detecting (4031) whether the motion state parameter is a fallen state parameter, which indicates that the acceleration is the gravity acceleration and the speed is not 0 during a first time period while the acceleration and the speed are both 0 during a second time period, wherein the first time period and the second time period are two consecutive time periods.

As used herein, the term "fallen state" refers to a parameter which indicates whether the wearable device is no longer being worn by the wearer because it has fallen or is in the process of falling, and/or is otherwise no longer being worn by the user.

The motion state parameter includes speed and acceleration, and the determining whether the wearable device is in the fallen state based on the motion state parameter may include: when the motion state parameter is the fallen state parameter, determining that the wearable device is in the fallen state; and when the motion state parameter is not the fallen state parameter, determining that the wearable device is not in the fallen state.

The sending information about falling to the terminal when the wearable device is in the fallen state may include: when the wearable device is in the fallen state, acquiring a temperature of the location where the wearable device contacts with a user's skin at current time; determining whether the temperature of the location where the wearable device contacts with the user's skin at the current time is lower than the temperature of the location where the wearable device contacts with the user's skin at a previous time, wherein the difference between the current time and the previous time is t seconds, t>0; and when the temperature at the current time is lower than the temperature at the previous time, sending the information about falling to the terminal.

The information about falling may include at least one of the location of the wearable device and fallen time of the wearable device. The sending the information about falling to the terminal may include: determining the location of the wearable device; and/or setting the time when the speed and the acceleration of the wearable device both become 0 as the fallen time of the wearable device.

Optionally, the method may further include sending out a warning signal.

The warning signal may include at least one of sound signal, vibration signal and light signal.

According to a second aspect of embodiments of the present disclosure, a communication method for a wearable device is provided, including: receiving information about falling sent by a wearable device, wherein the information about falling was sent when the wearable device determined the wearable device was in a fallen state based on an acquired motion state parameter of the wearable device; and sending an alerting signal based on the information about falling.

The information about falling may include at least one of the location of the wearable device and fallen time of the wearable device, and the method may further include displaying the information about falling.

The method may further include: determining a location of a terminal; and displaying the location of the terminal.

According to a third aspect of embodiments of the present disclosure, a communication apparatus for a wearable device is provided, including: an acquisition module configured to acquire a motion state parameter of a wearable device, the motion state parameter comprising speed and acceleration; a determination module configured to detect, at a current time, whether a temperature of a location where the wearable device contacts with a user's skin is higher than a temperature of another location of the wearable device, and, if the temperature of the location where the wearable device contacts with the user's skin is not higher than the temperature of the other location of the wearable device, to determine whether the wearable device is in a fallen state, in which the wearable device is no longer being worn because it has fallen, the determination being based on the motion state parameter within a predefined time period before the current time; and if the wearable device is in the fallen state, a sending module is configured to send information about falling to a terminal to instruct the terminal to send out an alerting signal when the wearable device is in the fallen state, wherein the determination module is configured to detect whether the motion state parameter is a fallen state parameter, which indicates that the acceleration is the gravity acceleration and the speed is not 0 during a first time period while the acceleration and the speed are both 0 during a second time period, wherein the first time period and the second time period are two consecutive time periods.

The motion state parameter includes speed and acceleration, and the determination module may be configured to: when the motion state parameter is the fallen state parameter, determine that the wearable device is in the fallen state; and when the motion state parameter is not the fallen state parameter, determine that the wearable device is not in the fallen state.

The sending module may be configured to: when the wearable device is in the fallen state, acquire a temperature of the location where the wearable device contacts with a user's skin at current time; determine whether the temperature of the location where the wearable device contacts with the user's skin at the current time is lower than the temperature of the location where the wearable device contacts with the user's skin at a previous time, wherein the difference between the current time and the previous time is t seconds, t>0; and when the temperature at the current time is lower than the temperature at the previous time, send the information about falling to the terminal.

The information about falling may include at least one of the location of the wearable device and fallen time of the wearable device, and the sending module may be configured to: determine the location of the wearable device; and/or set the time when the speed and the acceleration of the wearable device both become 0 as the fallen time of the wearable device.

The apparatus may further include a warning module configured to send out a warning signal. The warning signal may include at least one of sound signal, vibration signal and light signal.

According to a fourth aspect of embodiments of the present disclosure, a communication apparatus for a wearable device is provided, including: a receiving module configured to receive information about falling sent by a wearable device, wherein the information about falling was sent when the wearable device determined the wearable device was in a fallen state based on an acquired motion state parameter of the wearable device; and an alerting module configured to send an alerting signal based on the information about falling.

The information about falling may include at least one of the location of the wearable device and fallen time of the wearable device, and the apparatus may further include a first display module configured to display the information about falling.

The apparatus may further include: a determination module configured to determine a location of a terminal; and a second display module configured to display the location of the terminal.

Where functional modules are referred to in apparatus embodiments for carrying out various steps of the described method(s) it will be understood that these modules may be implemented in hardware, in software, or a combination of the two. When implemented in hardware, the modules may be implemented as one or more hardware modules, such as one or more application specific integrated circuits. When implemented in software, the modules may be implemented as one or more computer programs that are executed on one or more processors.

According to a fifth aspect of embodiments of the present disclosure, a communication apparatus for a wearable device is provided, including a processor and a memory for storing processor-executable instructions, wherein the processor is configured to: acquire a motion state parameter of a wearable device; determine whether the wearable device is in a fallen state based on the motion state parameter; and send information about falling to a terminal to instruct the terminal to send out an alerting signal when the wearable device is in the fallen state.

According to a sixth aspect of embodiments of the present disclosure, a communication apparatus for a wearable device is provided, including a processor and a memory for storing processor-executable instructions, wherein the processor is configured to: receive information about falling sent by a wearable device, wherein the information about falling was sent when the wearable device determined the wearable device was in a fallen state based on an acquired motion state parameter of the wearable device; and send an alerting signal based on the information about falling.

According to a seventh aspect of embodiments of the present disclosure, a communication system for a wearable device is provided, including a wearable device and a terminal, wherein the wearable device includes the communication apparatus for wearable device of the third aspect and the terminal includes the communication apparatus for wearable device of the fourth aspect.

In the communication method, apparatus and system for wearable device provided by the disclosure, the wearable device may send information about falling to a terminal to instruct the terminal to send out an alerting signal when determining that the wearable device is in a fallen state based on an acquired motion state parameter. Therefore, the communication content between the wearable device and the terminal may include the information about falling in addition to the data on health and exercise, and thus the communication content between the wearable device and the terminal can be expanded.

It is to be understood that both the forgoing general description and the following detailed description are exemplary only, and are not restrictive of the present disclosure.

### BRIEF DESCRIPTION OF THE DRAWINGS

In order to describe the embodiments of the disclosure clearly, below is a brief introduction of the accompanying drawings used in the description of the embodiments. Obviously, the drawings are only some embodiments of the disclosure. For those ordinary skilled in the art, other drawings are also possible based on these drawings without creative labor.
Fig. 1 is a schematic diagram illustrating an environment in which a communication method for wearable device may be implemented according to an exemplary embodiment.
Fig. 2 is a flow diagram illustrating a communication method for wearable device according to an exemplary embodiment.
Fig. 3 is a flow diagram illustrating another communication method for wearable device according to an exemplary embodiment.
Fig. 4-1 is a flow diagram illustrating yet another communication method for wearable device according to an exemplary embodiment.
Fig. 4-2 is a flow diagram illustrating a method for determining whether a wearable device is in a fallen state according to an exemplary embodiment.
Fig. 4-3 is a schematic diagram of displaying the information about falling on a terminal according to an exemplary embodiment.
Fig. 4-4 is a schematic diagram of displaying the location of the terminal on the terminal according to an exemplary embodiment.
Fig. 5-1 is a block diagram illustrating a communication apparatus for wearable device according to an exemplary embodiment.
Fig. 5-2 is a block diagram illustrating another communication apparatus for wearable device according to an exemplary embodiment.
Fig. 6-1 is a block diagram illustrating yet another communication apparatus for wearable device according to an exemplary embodiment.
Fig. 6-2 is a block diagram illustrating yet another communication apparatus for wearable device according to an exemplary embodiment.
Fig. 7 is a block diagram illustrating yet another communication apparatus for wearable device according to an exemplary embodiment.
Fig. 8 is a block diagram illustrating yet another communication apparatus for wearable device according to an exemplary embodiment.

The drawings herein are incorporated in the specification and form a part of the specification. They conform to the embodiments of the present disclosure and are used to explain the principles of the present disclosure along with the specification.

### DETAILED DESCRIPTION

In order to make the objects, technical solutions and advantages of the disclosure clear, the disclosure may be described further in detail in connection with the accompanying drawings below. Obviously, the described embodiments are only a portion of the embodiments of the disclosure, not all embodiments. Based on the embodiments in the disclosure, all other embodiments made by those ordinary skilled in the art without creative labor belong to the scope of the disclosure.

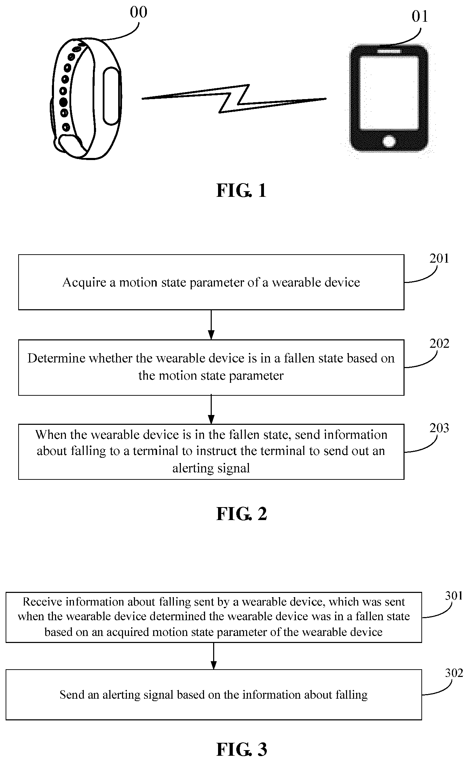

Fig. 1 is a schematic diagram illustrating an environment in which a communication method for a wearable device may be implemented according to an exemplary embodiment.

The environment may include a wearable device 00 and a terminal 01. The wearable device 00 may be an intelligent bracelet, an intelligent watch, intelligent glasses, and the like. The terminal 01 may be a smart phone, a computer, a multimedia player, and an ebook reader, etc.

The wearable device 00 and the terminal 01 can be connected by a wireless network. Fig. 2 is a flow diagram illustrating a communication method for a wearable device which may be applied in the wearable device 00 shown in Fig. 1 according to an exemplary embodiment. As illustrated in Fig. 2, the method may include the following steps.

In step 201, a motion state parameter of a wearable device is acquired.

In step 202, it is determined whether the wearable device is in a fallen state, based on the motion state parameters.

In step 203, when the wearable device is in the fallen state, information about falling is sent to a terminal to instruct the terminal to send out an alerting signal.

In summary, in the communication method for a wearable device provided by the embodiment, the wearable device may send information about falling to a terminal to instruct the terminal to send out an alerting signal when determining the wearable device is in the fallen state based on the acquired motion state parameter. Therefore, the communication content between the wearable device and the terminal may include the information about falling in addition to the data on health and exercise, and thus the communication content between the wearable device and the terminal can be expanded.

The motion state parameter may include speed and acceleration, and the determining whether the wearable device is in the fallen state based on the motion state parameter may include: detecting whether the motion state parameter is a fallen state parameter, which indicates that during a first time period the acceleration is the gravity acceleration and the speed is not 0 while during a second time period the acceleration and the speed are both 0, wherein the first time period and the second time period are two consecutive time periods; when the motion state parameter is the fallen state parameter, determining that the wearable device is in the fallen state; and when the motion state parameter is not the fallen state parameter, determining that the wearable device is not in the fallen state.

The sending information about falling to the terminal when the wearable device is in the fallen state may include: when the wearable device is in the fallen state, acquiring a temperature of the location where the wearable device contacts with a user's skin at current time; determining whether the temperature of the location where the wearable device contacts with the user's skin at the current time is lower than the temperature of the location where the wearable device contacts with the user's skin at a previous time, wherein the difference between the current time and the previous time is t seconds, t>0; and when the temperature at the current time is lower than the temperature at the previous time, sending information about falling to the terminal.

The determining whether the wearable device is in the fallen state may include: detecting whether a temperature of the location where the wearable device contacts with the user's skin is higher than a temperature of another location of the wearable device; and when the temperature of the location where the wearable device contacts with the user's skin is not higher than the temperature of the other location of the wearable device, determining whether the wearable device is in the fallen state.

The information about falling may include at least one of the location of the wearable device and fallen time of the wearable device. The sending information about falling to the terminal may include: determining the location of the wearable device; and/or setting the time when the speed and the acceleration of the wearable device both become 0 as the fallen time of the wearable device.

Optionally, the method may further include sending out a warning signal.

The warning signal may include at least one of sound signal, vibration signal and light signal.

In summary, in the communication method for a wearable device provided by the embodiment, the wearable device may send information about falling to a terminal when determining the wearable device is in a fallen state based on an acquired motion state parameter. Therefore, the communication content between the wearable device and the terminal may include the information about falling in addition to the data on health and exercise, and thus the communication content between the wearable device and the terminal can be expanded. In the fallen state, the wearable device can send out a warning signal, effectively reducing the chance of loss of the wearable device.

Fig. 3 is a flow diagram illustrating a communication method for a wearable device which may be applied in the terminal 01 shown in Fig. 1 according to an exemplary embodiment. As illustrated in Fig. 3, the method may include the following steps.

In step 301, information about falling sent by a wearable device is received, wherein the information about falling was sent when the wearable device determined the wearable device was in a fallen state based on an acquired motion state parameter of the wearable device.

In step 302, an alerting signal is sent based on the information about falling.

In summary, in the communication method for a wearable device provided by the embodiment, the information sent by the wearable device which is received by the terminal may include the information about falling sent by the wearable device in addition to the data of health and exercise, and thus the communication content between the wearable device and the terminal can be expanded. Upon receiving the information about falling, the terminal may send an alerting signal to inform the user of the falling of the wearable device, effectively reducing the chance of loss of the wearable device.

The information about falling may include at least one of the location of the wearable device and the fallen time of the wearable device. The method may further include displaying the information about falling.

The method may further include determining the location of the terminal and displaying the location of the terminal.

In summary, in the communication method for a wearable device provided by the embodiment, the information sent by the wearable device which is received by the terminal may include the information about falling sent by the wearable device in addition to the data on health and exercise, and thus the communication content between the wearable device and the terminal can be expanded. Upon receiving the information about falling, the terminal may send an alerting signal to inform the user of the falling of the wearable device, effectively reducing the chance of loss of the wearable device.

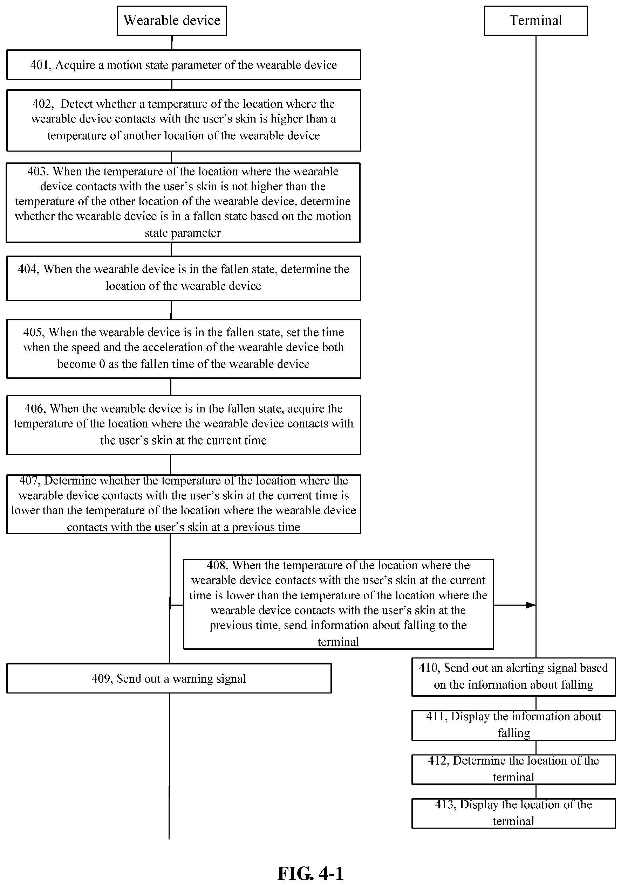

Fig. 4-1 is a flow diagram illustrating another communication method for wearable device which may be applied in the implementation environment shown in Fig. 1 according to an exemplary embodiment. As illustrated in Fig. 4-1, the method may include the following steps.

In step 401, a wearable device acquires a motion state parameter of the wearable device.

The motion state parameter may include speed and acceleration. In an exemplary embodiment, the wearable device may be equipped with a motion sensor, such as accelerometer or gyroscope, etc., which can acquire the motion state parameter such as speed and acceleration of the wearable device in real time. For example, the wearable device may be a bracelet, and the motion state parameter of the bracelet acquired by the motion sensor in the bracelet at a current time 8:00 may be: magnitude of speed: 4.5 m/s; direction of speed: vertically downward; magnitude of acceleration: 9.8 m/s²; direction of acceleration: vertically downward. Here the "vertically downward" is the direction of gravity, i.e., the direction of the gravity acceleration.

In step 402, the wearable device detects whether a temperature of the location where the wearable device contacts with the user's skin is higher than a temperature of another location of the wearable device.

In an exemplary embodiment, the wearable device may be equipped with at least two temperature sensors. The at least two temperature sensors may be located respectively at a location where the wearable device contacts with the user's skin and another location where the wearable device does not contact with the user's skin. For example, if the wearable device is an intelligent bracelet, the location where the intelligent bracelet contacts with the user's skin may be the inner side of the intelligent bracelet; if the wearable device is an intelligent watch, the location where the intelligent watch contacts with the user's skin may be the inner side of the watch band of the intelligent watch; if the wearable device is a pair of intelligent glasses, the location where the pair of intelligent glasses contacts with the user's skin may be the inner side of the legs of the intelligent glasses or at the nose pad of the intelligent glasses. The wearable device can acquire the temperatures detected by the at least two temperature sensors in real time and detect whether the temperature of the location where the wearable device contacts with the user's skin is higher than the temperature of the other location of the wearable device. If the temperature of the location where the wearable device contacts with the user's skin is higher than the temperature of the other location of the wearable device, the wearable device may determine that the user is wearing the wearable device; if the temperature of the location where the wearable device contacts with the user's skin is not higher than the temperature of the other location of the wearable device, the wearable device may determine that the user is not wearing the wearable device. For example, assuming that the wearable device is a bracelet, a temperature sensor 1 is provided on the inner side of the bracelet (i.e., the location where the wearable device contacts with the user's skin), and a temperature sensor 2 is provided on the outer side of the bracelet (i.e., the other location where the wearable device does not contact with the user's skin), the bracelet can acquire the temperatures detected by the two temperature sensors in real time and compare to determine whether the temperature detected by the temperature sensor 1 is higher than the temperature detected by the temperature sensor 2.

In step 403, when the temperature of the location where the wearable device contacts with the user's skin is not higher than the temperature of the other location of the wearable device, the wearable device determines whether the wearable device is in a fallen state based on the motion state parameter.

When the temperature of the location where the wearable device contacts with the user's skin is not higher than the temperature of the other location of the wearable device, the wearable device may determine that the user is not wearing the wearable device at the current time. The wearable device can acquire the motion state parameter within a predefined time period from the current time and determine whether the wearable device is in the fallen state based on the motion state parameter. The predefined time period may be 10 seconds or 1 minute, and is not limited in the embodiments. Assuming the wearable device is a bracelet, and the bracelet acquires the temperatures detected by the two sensors at 8:01 of 27 degrees at the temperature sensor 1 and 27 degrees at the temperature sensor 2, because the temperature of the location where the bracelet contacts with the user's skin is not higher than the temperature of the other location of the bracelet, the bracelet may acquire the motion state parameter within 10 seconds immediately before the current time 8:01 and determine whether the bracelet is in the fallen state based on the motion state parameter within the 10 seconds.

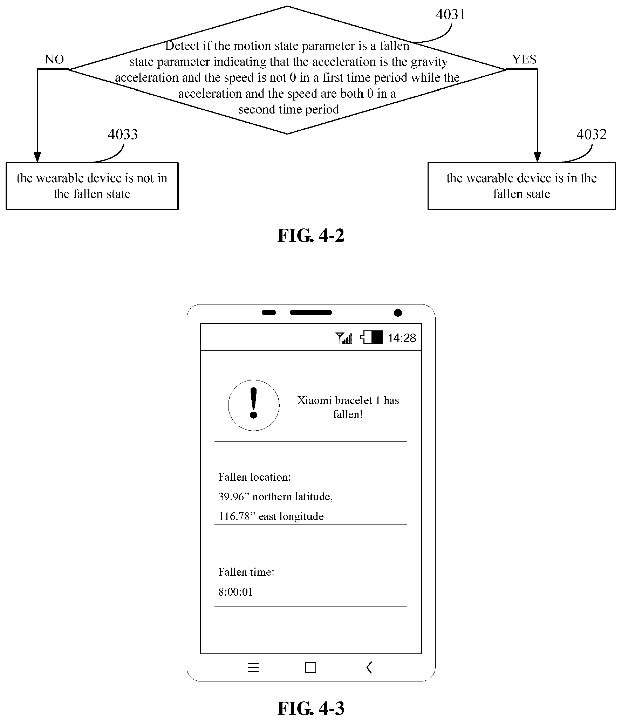

Fig. 4-2 is a flow diagram illustrating a method for determining whether the bracelet is in the fallen state according to an exemplary embodiment. As illustrated in Fig. 4-2, the method may include the following steps.

In step 4031, it is detected whether the motion state parameter is a fallen state parameter. The fallen state parameter may be: during a first time period the acceleration is the gravity acceleration and the speed is not 0; while during a second time period the acceleration and the speed are both 0.

The first time period and the second time period may be any two consecutive time periods. When the acceleration is 9.8 m/s² vertically downward, the wearable device may determine that the acceleration is the gravity acceleration. When the motion state parameter is the fallen state parameter, the step 4032 is performed; when the motion state parameter is not the fallen state parameter, the step 4033 is performed. In an exemplary embodiment, the wearable device can acquire the motion state parameter of the wearable device in real time and detect whether the motion state parameters within any two consecutive time periods satisfy the above-mentioned condition on the fallen state parameters.

In step 4032, it is determined that the wearable device is in the fallen state.

When the acceleration of the wearable device is the gravity acceleration and the speed is not 0 in the first time period, the wearable device may be in a falling state. When the acceleration and the speed of the wearable device are both 0 in the second time period which is consecutive with the first time period, the wearable device may be in a falling-stopped state. The first time period and the second time period may be any two consecutive time periods. When the motion state parameters of the wearable device within the two consecutive time periods satisfy the above-mentioned fallen state parameters, the wearable device may determine that the wearable device is in the fallen state. For example, assuming the wearable device is a bracelet. The motion state parameters acquired by the bracelet at 8:00 may be: magnitude of speed: 4.5 m/s; direction of speed: vertically downward; magnitude of acceleration: 9.8 m/s²; direction of acceleration: vertically downward, the bracelet may determine that the acceleration is the gravity acceleration. The bracelet may detect that the acceleration of the bracelet is always the gravity acceleration and the speed is not 0 within the next 1 second after 8:00, and the acceleration and the speed of the bracelet are both 0 during 8:00:01-8:00:03. Then the bracelet may determine that the bracelet is in the fallen state, where the first time period is 8:00:00-8:00:01 and the second time period is 8:00:01-8:00:02.

It should be noted that in practical application, the first time period may be the time period when the wearable device is free falling and the second time period may be the time period after the wearable device stops free falling. Therefore, when the wearable device falls from a higher location, the first time period may be longer. The lengths of the first time period and the second time period are not limited in the present disclosure.

In step 4033, it is determined that the wearable device is not in the fallen state.

When the motion state parameter of the wearable device is not the fallen state parameter, the wearable device may determine that the wearable device is not in the fallen state. If the motion state parameter of the wearable device does not satisfy any one of the conditions in the fallen state parameter indicated in step 4031, the wearable device may determine that the wearable device is not in the fallen state.

In step 404, when the wearable device is in the fallen state, the wearable device determines the location of the wearable device.

In an exemplary embodiment, the wearable device may be equipped with a positioning module. When the wearable device is determined to be in the fallen state, the wearable device may determine the current location of the wearable device through the positioning module. For example, the location determined by the wearable device may be the longitude and latitude of the location, such as 39.96" northern latitude, 116.78" east longitude, or may be detailed road information of the location, such as No. xx, Wangjing West Road, Chaoyang District, Beijing.

In step 405, when the wearable device is in the fallen state, the wearable device sets the time when the speed and the acceleration of the wearable device both become 0 as the fallen time of the wearable device.

The time when the speed and acceleration of the wearable device both become 0 is the time when the wearable device reaches the ground, and therefore the wearable device may take that time as the fallen time of the wearable device. For example, assuming that the time when the speed and acceleration of the bracelet both became 0 at 8:00:01, the bracelet may take the time 8:00:01 as the fallen time of the bracelet.

In step 406, when the wearable device is in fallen state, the wearable device acquires the temperature of the location where the wearable device contacts with a user's skin at the current time.

In an exemplary embodiment, when the wearable device determines that the wearable device is in the fallen state based on the motion state parameter, in order to ensure the accuracy of this result, the wearable device may also acquire the temperature of the location where the wearable device contacts with the user's skin at the current time. For example, assuming the wearable device is a bracelet, which is equipped with a temperature sensor 1 on the inner side, and the bracelet determines at 8:00:02 that the bracelet is in the fallen state, the bracelet may acquire the temperature of 33 degrees detected by the temperature sensor 1 at the current time 8:00:02.

In step 407, the wearable device determines whether the temperature of the location where the wearable device contacts with the user's skin at the current time is lower than the temperature of the location where the wearable device contacts with the user's skin at a previous time. The difference between the current time and the previous time is t seconds, t>0.

For example, assuming that t is 2 seconds and the current time is 8:00:02, the bracelet may acquire the temperature of the location where the bracelet contacts with the user's skin at the previous time 8:00, which may be 35 degrees.

In step 408, when the temperature of the location where the wearable device contacts with the user's skin at the current time is lower than the temperature of the location where the wearable device contacts with the user's skin at the previous time, the wearable device sends information about falling to a terminal.

When the wearable device is in the fallen state and the temperature of the location where the wearable device contacts with the user's skin at the current time is lower than the temperature of the location where the wearable device contacts with the user's skin at the previous time, the wearable device may determine the temperature of the location where the wearable device contacts with the user's skin has dropped and send the information about falling to the terminal to instruct the terminal to send out an alerting signal. The information about falling may include at least one of the location of the wearable device and the fallen time of the wearable device. By determining the wearable device is in the fallen state based on the motion state parameter and determining the wearable device has fallen based on the drop in the temperature of the location where the wearable device contacts with the user's skin, the likelihood that the wearable device sends the information about falling in error may be reduced, thus improving the accuracy of the information about falling sent by the wearable device. For example, since the temperature 33°C of the location where the bracelet contacts with the user's skin at the current time 8:00:02 is lower than the temperature 35°C of the location where the wearable device contacts with the user's skin at the previous time 8:00, the bracelet may send the information about falling to the terminal. The information may be: location: 39.96" northern latitude, 116.78" east longitude; fallen time: 8:00:01.

In step 409, the wearable device sends out a warning signal.

Upon determined to be in the fallen state, the wearable device may also send out a warning signal. The warning signal may include at least one of sound signal, vibration signal and light signal. Sending out the warning signal by the wearable device may inform the user that the wearable device has fallen, thus effectively reducing the chance of loss of the wearable device.

In step 410, the terminal sends out an alerting signal based on the information about falling.

The alerting signal may include at least one of sound signal, vibration signal and light signal. Upon receiving the information about falling, the terminal may send out the alerting signal. The alerting signal may inform the user that the wearable device has fallen, thus reducing the chance of loss of the wearable device.

In step 411, the terminal displays the information about falling.

The terminal may display an identification of the wearable device as well as displaying the information about falling sent by the wearable device. The information about falling may include at least one of the location of the wearable device and the fallen time of the wearable device. Displaying the information about falling may facilitate the user's finding the fallen wearable device, thus reducing the chance of loss of the wearable device. Fig. 4-3 is a schematic diagram of displaying the information about falling on the terminal according to an exemplary embodiment. Assuming the information about falling sent by the bracelet which is received by the terminal is that the location is 39.96" northern latitude, 116.78" east longitude and the fallen time is 8:00:01, and the identification of the bracelet is Xiaomi bracelet 1, the terminal may display in the interface shown in Fig. 4-3: Xiaomi bracelet 1 has fallen! Fallen location: 39.96" northern latitude, 116.78" east longitude; Fallen time: 8:00:01.

In step 412, the terminal determines the location of the terminal.

In an exemplary embodiment, upon receiving the information about falling, the terminal may also determine the location of the terminal at the current time with a positioning module in the terminal. For example, the location determined by the terminal at the current time may be 39.97" northern latitude, 116.78" east longitude.

In step 413, the terminal displays the location of the terminal.

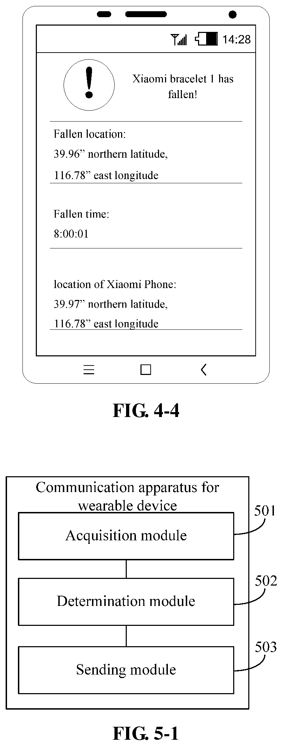

Fig. 4-4 is a schematic diagram of displaying the location of the terminal on the terminal according to an exemplary embodiment. As shown in Fig. 4-4, assuming the terminal is a Xiaomi Phone, the content displayed on the terminal may be: location of Xiaomi Phone: 39.97" northern latitude, 116.78" east longitude, so that if the information about falling sent by the wearable device received by the terminal is delayed, the terminal may determine a distance from the location where the wearable device fell based on the current location to help the user find the fallen wearable device.

It should be noted that in the communication method for a wearable device provided in the embodiment, the order of the steps can be changed, some of the steps can be removed, or additional steps may be added as required. For example, the steps 402, 406 and 407 can be removed as required, and the steps 409 and 410 can be performed simultaneously. Any variations that may be recognized by those skilled in the art in the technical scope disclosed in the disclosure should be included in the scope of the disclosure, and will not be elaborated herein.

In summary, in the communication method for a wearable device provided by the embodiment, the wearable device may send information about falling to a terminal when determining the wearable device is in a fallen state based on the acquired motion state parameter. Therefore, the communication content between the wearable device and the terminal may include the information about falling in addition to the data on health and exercise, and thus the communication content between the wearable device and the terminal can be expanded. When in the fallen state, the wearable device can send out a warning signal, thus effectively reducing the chance of loss of the wearable device.

Fig. 5-1 is a block diagram illustrating a communication apparatus for a wearable device according to an exemplary embodiment. As shown in Fig. 5-1, the apparatus includes: an acquisition module 501 configured to acquire a motion state parameter of a wearable device; a determination module 502 configured to determine whether the wearable device is in a fallen state based on the motion state parameter; and a sending module 503 configured to send information about falling to a terminal to instruct the terminal to send out an alerting signal when the wearable device is in the fallen state.

In summary, in the communication apparatus for a wearable device provided by the embodiment, the wearable device may send information about falling to a terminal when determining the wearable device is in a fallen state based on the acquired motion state parameter. Therefore, the communication content between the wearable device and the terminal may include the information about falling in addition to the data on health and exercise, and thus the communication content between the wearable device and the terminal can be expanded. When in the fallen state, the wearable device can send out a warning signal, thus effectively reducing the chance of loss of the wearable device.

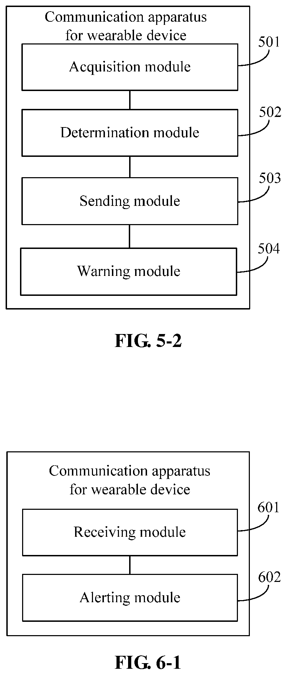

Fig. 5-2 is a block diagram illustrating a communication apparatus for a wearable device according to an exemplary embodiment. As shown in Fig. 5-2, the apparatus includes: an acquisition module 501 configured to acquire a motion state parameter of a wearable device; a determination module 502 configured to determine whether the wearable device is in a fallen state based on the motion state parameters; a sending module 503 configured to send information about falling to a terminal to instruct the terminal to send out alerting signals when the wearable device is in the fallen state; and a warning module 504 configured to send out a warning signal.

The motion state parameter may include speed and acceleration, and the determination module 502 may be configured to: detect whether the motion state parameter is a fallen state parameter, which indicates that the acceleration is the gravity acceleration and the speed is not 0 during a first time period while the acceleration and the speed are both 0 during a second time period, wherein the first time period and the second time period are any two consecutive time periods; when the motion state parameter is the fallen state parameter, determine that the wearable device is in the fallen state; and when the motion state parameter is not the fallen state parameter, determine that the wearable device is not in the fallen state.

The sending module 503 may be configured to: acquire, when the wearable device is in the fallen state, a temperature of the location where the wearable device contacts with a user's skin at current time; determine whether the temperature of the location where the wearable device contacts with the user's skin at the current time is lower than the temperature of the location where the wearable device contacts with the user's skin at a previous time, wherein the difference between the current time and the previous time is t seconds, t>0; and when the temperature at the current time is lower than the temperature at the previous time, send the information about falling to the terminal.

The determination module 502 may be configured to: detect whether a temperature of the location where the wearable device contacts with the user's skin is higher than a temperature of another location of the wearable device; and when the temperature of the location where the wearable device contacts with the user's skin is not higher than the temperature of the other location of the wearable device, determine whether the wearable device is in the fallen state.

The information about falling may include at least one of the location of the wearable device and the fallen time of the wearable device. The sending module 503 may be configured to: determine the location of the wearable device; and/or set the time when the speed and the acceleration of the wearable device both become 0 as the fallen time of the wearable device.

The warning signal may include at least one of sound signal, vibration signal and light signal.

In summary, in the communication apparatus for a wearable device provided by the disclosure, the wearable device may send information about falling to a terminal when determining the wearable device is in a fallen state based on the acquired motion state parameter. Therefore, the communication content between the wearable device and the terminal may include the information about falling in addition to the data on health and exercise, and thus the communication content between the wearable device and the terminal can be expanded. When in the fallen state, the wearable device can send out a warning signal, thus effectively reducing the chance of loss of the wearable device.

Fig. 6-1 is a block diagram illustrating another communication apparatus for a wearable device according to an exemplary embodiment. As shown in Fig. 6-1, the apparatus includes: a receiving module 601 configured to receive information about falling sent by a wearable device, wherein the information about falling was sent when the wearable device determined the wearable device was in a fallen state based on an acquired motion state parameter of the wearable device; and an alerting module 602 configured to send an alerting signal based on the information about falling.

In summary, in the communication apparatus for a wearable device provided by the disclosure, the information sent by the wearable device which is received by the terminal may include the information about falling sent by the wearable device in addition to the data on health and exercise, and thus the communication content can be expanded between the wearable device and the terminal. Upon receiving the information about falling, the terminal may send an alerting signal to inform the user the falling of the wearable device, thus effectively reducing the chance of loss of the wearable device.

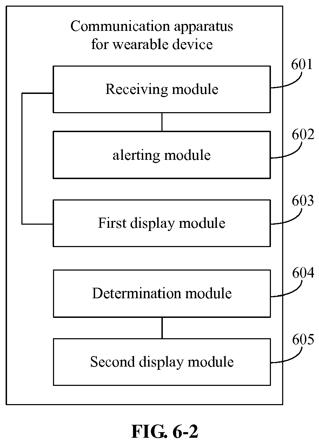

Fig. 6-2 is a block diagram illustrating another communication apparatus for a wearable device according to an exemplary embodiment. As shown in Fig. 6-2, the apparatus includes: a receiving module 601 configured to receive information about falling sent by a wearable device, wherein the information about falling was sent when the wearable device determined the wearable device was in a fallen state based on an acquired motion state parameter of the wearable device; an alerting module 602 configured to send an alerting signal based on the information about falling; a first display module 603 configured to display the information about falling, wherein the information about falling may include at least one of the location of the wearable device and the fallen time of the wearable device; a determination module 604 configured to determine a location of a terminal; and a second display module 605 configured to display the location of the terminal.

In summary, in the communication apparatus for a wearable device provided by the disclosure, the information sent by the wearable device which is received by the terminal may include the information about falling sent by the wearable device in addition to the data on health and exercise, and thus the communication content between the wearable device and the terminal can be expanded. Upon receiving the information about falling, the terminal may send an alerting signal to inform the user the falling of the wearable device, thus effectively reducing the chance of loss of the wearable device.

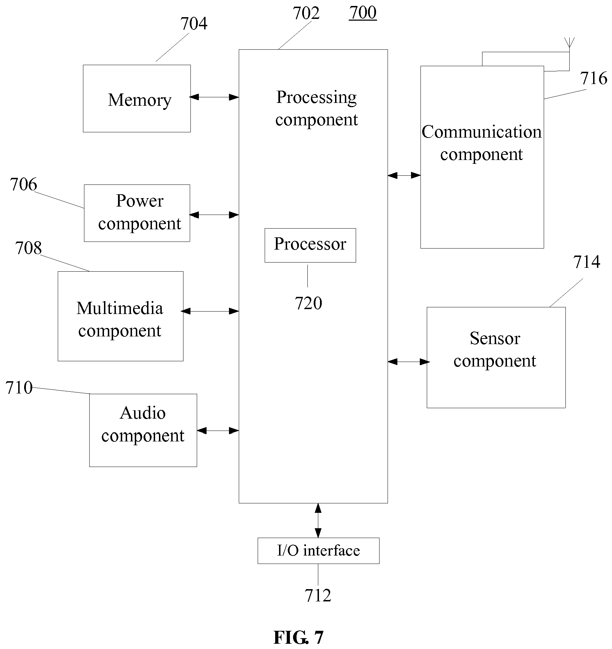

Fig. 7 is a block diagram of an apparatus 700 for a wearable device according to an exemplary embodiment. For example, the apparatus 700 may be a mobile phone, a computer, a digital broadcast terminal, a messaging device, a gaming console, a tablet, a medical device, exercise equipment, a personal digital assistant, an intelligent bracelet, an intelligent watch and the like.

Referring to Fig. 7, the apparatus 700 may include one or more of the following components: a processing component 702, a memory 704, a power component 706, a multimedia component 708, an audio component 710, an input/output (I/O) interface 712, a sensor component 714, and a communication component 716.

The processing component 702 typically controls overall operations of the apparatus 700, such as the operations associated with display, telephone calls, data communications, camera operations, and recording operations. The processing component 702 may include one or more processors 720 to execute instructions to perform all or part of the steps in the above described methods. Moreover, the processing component 702 may include one or more modules which facilitate the interaction between the processing component 702 and other components. For instance, the processing component 702 may include a multimedia module to facilitate the interaction between the multimedia component 708 and the processing component 702.

The memory 704 is configured to store various types of data to support the operation of the apparatus 700. Examples of such data include instructions for any applications or methods operated on the apparatus 700, contact data, phonebook data, messages, pictures, video, etc. The memory 704 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 706 provides power to various components of the apparatus 700. The power component 706 may include a power management system, one or more power sources, and any other components associated with the generation, management, and distribution of power for the apparatus 700.

The multimedia component 708 includes a screen providing an output interface between the apparatus 700 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 708 includes a front camera and/or a rear camera. The front camera and the rear camera may receive an external multimedia datum while the apparatus 700 is in an operation mode, such as a photographing mode or a video mode. Each of the front camera and the rear camera may be a fixed optical lens system or have optical focusing and zooming capability.

The audio component 710 is configured to output and/or input audio signals. For example, the audio component 710 includes a microphone ("MIC") configured to receive an external audio signal when the apparatus 700 is in an operation mode, such as a call mode, a recording mode, and a voice recognition mode. The received audio signal may be further stored in the memory 704 or transmitted via the communication component 716. In some embodiments, the audio component 710 further includes a speaker to output audio signals.

The I/O interface 712 provides an interface between the processing component 702 and peripheral interface modules, the peripheral interface modules being, for example, a keyboard, a click wheel, buttons, and the like. The buttons may include, but are not limited to, a home button, a volume button, a starting button, and a locking button.

The sensor component 714 includes one or more sensors to provide status assessments of various aspects of the apparatus 700. For instance, the sensor component 714 may detect an open/closed status of the apparatus 700, relative positioning of components (e.g., the display and the keypad, of the apparatus 700), a change in position of the apparatus 700 or a component of the apparatus 700, a presence or absence of user contact with the apparatus 700, an orientation or an acceleration/deceleration of the apparatus 700, and a change in temperature of the apparatus 700. The sensor component 714 may include a proximity sensor configured to detect the presence of a nearby object without any physical contact. The sensor component 714 may also include a light sensor, such as a CMOS or CCD image sensor, for use in imaging applications. In some embodiments, the sensor component 714 may also include an accelerometer sensor, a gyroscope sensor, a magnetic sensor, a pressure sensor, or a temperature sensor.

The communication component 716 is configured to facilitate communication, wired or wirelessly, between the apparatus 700 and other devices. The apparatus 700 can access a wireless network based on a communication standard, such as WiFi, 2G, or 3G, or a combination thereof. In an exemplary embodiment, the communication component 716 receives a broadcast signal or broadcast associated information from an external broadcast management system via a broadcast channel. In an exemplary embodiment, the communication component 716 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the apparatus 700 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer-readable storage medium including instructions, such as included in the memory 704, executable by the processor 720 in the apparatus 700, for performing the above-described methods. For example, the non-transitory computer-readable storage medium may be a ROM, a RAM, a CD-ROM, a magnetic tape, a floppy disc, an optical data storage device, and the like.

The disclosure proides a non-transitory computer readable storage medium having stored therein instructions that, when executed by the processor of the apparatus 700, cause the apparatus 700 to perform a communication method for wearable device, including: acquiring a motion state parameter of a wearable device; determining whether the wearable device is in a fallen state based on the motion state parameter; and sending information about falling to a terminal to instruct the terminal to send out an alerting signal when the wearable device is in the fallen state.

The motion state parameter may include speed and acceleration and the determining whether the wearable device is in the fallen state based on the motion state parameter may include: detecting whether the motion state parameter is a fallen state parameter, which indicates that the acceleration is the gravity acceleration and the speed is not 0 during a first time period while the acceleration and the speed are both 0 during a second time period, wherein the first time period and the second time period are two consecutive time periods; when the motion state parameter is the fallen state parameters, determining that the wearable device is in the fallen state; and when the motion state parameter is not the fallen state parameter, determining that the wearable device is not in the fallen state.

The sending information about falling to the terminal when the wearable device is in the fallen state may include: when the wearable device is in the fallen state, acquiring a temperature of the location where the wearable device contacts with a user's skin at current time; determining whether the temperature of the location where the wearable device contacts with the user's skin at the current time is lower than the temperature of the location where the wearable device contacts with the user's skin at a previous time, wherein the difference between the current time and the previous time is t seconds, t>0; and when the temperature at the current time is lower than the temperature at the previous time, sending the information about falling to the terminal.

The determining whether the wearable device is in the fallen state may include: detecting whether a temperature of the location where the wearable device contacts with the user's skin is higher than a temperature of another location of the wearable device; and when the temperature of the location where the wearable device contacts with the user's skin is not higher than the temperature of the other location of the wearable device, determining whether the wearable device is in the fallen state.

The information about falling may include at least one of the location of the wearable device and the fallen time of the wearable device. The sending the information about falling to the terminal may include: determining the location of the wearable device; and/or setting the time when the speed and the acceleration of the wearable device both become 0 as the fallen time of the wearable device.

The method may further include sending out a warning signal.

The warning signal may include at least one of sound signal, vibration signal and light signal.

In summary, in the communication apparatus for a wearable device provided by the disclosure, the wearable device may send information about falling to a terminal when determining the wearable device is in a fallen state based on the acquired motion state parameter. Therefore, the communication content between the wearable device and the terminal may include the information about falling in addition to the data on health and exercise, and thus the communication content between the wearable device and the terminal can be expanded. When in the fallen state, the wearable device can send out a warning signal, thus effectively reducing the chance of loss of the wearable device.

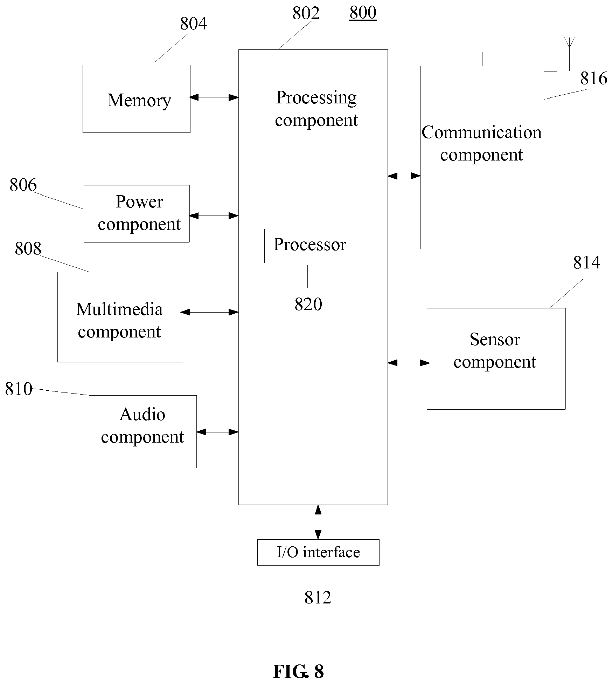

Fig. 8 is a block diagram of an apparatus 800 for a wearable device according to an exemplary embodiment. For example, the apparatus 800 may be a mobile phone, a computer, a digital broadcast terminal, a messaging device, a gaming console, a tablet, a medical device, exercise equipment, a personal digital assistant, and the like.

Referring to Fig. 8, the apparatus 800 may include one or more of the following components: a processing component 802, a memory 804, a power component 806, a multimedia component 808, an audio component 810, an input/output (I/O) interface 812, a sensor component 814, and a communication component 816.

The processing component 802 typically controls overall operations of the apparatus 800, such as the operations associated with display, telephone calls, data communications, camera operations, and recording operations. The processing component 802 may include one or more processors 820 to execute instructions to perform all or part of the steps in the above described methods. Moreover, the processing component 802 may include one or more modules which facilitate the interaction between the processing component 802 and other components. For instance, the processing component 802 may include a multimedia module to facilitate the interaction between the multimedia component 808 and the processing component 802.

The memory 804 is configured to store various types of data to support the operation of the apparatus 800. Examples of such data include instructions for any applications or methods operated on the apparatus 800, contact data, phonebook data, messages, pictures, video, etc. The memory 804 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 806 provides power to various components of the apparatus 800. The power component 806 may include a power management system, one or more power sources, and any other components associated with the generation, management, and distribution of power for the apparatus 800.

The multimedia component 808 includes a screen providing an output interface between the apparatus 800 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 808 includes a front camera and/or a rear camera. The front camera and the rear camera may receive an external multimedia datum while the apparatus 800 is in an operation mode, such as a photographing mode or a video mode. Each of the front camera and the rear camera may be a fixed optical lens system or have optical focusing and zooming capability.

The audio component 810 is configured to output and/or input audio signals. For example, the audio component 810 includes a microphone ("MIC") configured to receive an external audio signal when the apparatus 800 is in an operation mode, such as a call mode, a recording mode, and a voice recognition mode. The received audio signal may be further stored in the memory 804 or transmitted via the communication component 816. In some embodiments, the audio component 810 further includes a speaker to output audio signals.

The I/O interface 812 provides an interface between the processing component 802 and peripheral interface modules, the peripheral interface modules being, for example, a keyboard, a click wheel, buttons, and the like. The buttons may include, but are not limited to, a home button, a volume button, a starting button, and a locking button.

The sensor component 814 includes one or more sensors to provide status assessments of various aspects of the apparatus 800. For instance, the sensor component 814 may detect an open/closed status of the apparatus 800, relative positioning of components (e.g., the display and the keypad, of the apparatus 800), a change in position of the apparatus 800 or a component of the apparatus 800, a presence or absence of user contact with the apparatus 800, an orientation or an acceleration/deceleration of the apparatus 800, and a change in temperature of the apparatus 800. The sensor component 814 may include a proximity sensor configured to detect the presence of a nearby object without any physical contact. The sensor component 814 may also include a light sensor, such as a CMOS or CCD image sensor, for use in imaging applications. In some embodiments, the sensor component 814 may also include an accelerometer sensor, a gyroscope sensor, a magnetic sensor, a pressure sensor, or a temperature sensor.

The communication component 816 is configured to facilitate communication, wired or wirelessly, between the apparatus 800 and other devices. The apparatus 800 can access a wireless network based on a communication standard, such as WiFi, 2G, or 3G, or a combination thereof. In an exemplary embodiment, the communication component 816 receives a broadcast signal or broadcast associated information from an external broadcast management system via a broadcast channel. In an exemplary embodiment, the communication component 816 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the apparatus 800 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer-readable storage medium including instructions, such as included in the memory 804, executable by the processor 820 in the apparatus 800, for performing the above-described methods. For example, the non-transitory computer-readable storage medium may be a ROM, a RAM, a CD-ROM, a magnetic tape, a floppy disc, an optical data storage device, and the like.

The disclosure provides a non-transitory computer readable storage medium having stored therein instructions that, when executed by the processor of the apparatus 800, cause the apparatus 800 to perform a communication method for wearable device, including: receiving information about falling sent by a wearable device, wherein the information about falling was sent when the wearable device determined the wearable device was in a fallen state based on an acquired motion state parameter of the wearable device; and sending an alerting signal based on the information about falling.

The information about falling may include at least one of the location of the wearable device and the fallen time of the wearable device. The method may further include displaying the information about falling.

The method further includes determining a location of a terminal, and displaying the location of the terminal.

In summary, in the communication apparatus for a wearable device provided by the disclosure, the information sent by the wearable device which is received by the terminal may include the information about falling sent by the wearable device in addition to the data on health and exercise, and thus the communication content between the wearable device and the terminal can be expanded. Upon receiving the information about falling, the terminal may send an alerting signal to inform the user the falling of the wearable device, thus effectively reducing the chance of loss of the wearable device.

The term "and/or" used in the disclosure represents three possible relations between associated objects. For example, A and/or B may represent A, B, or A and B. In addition, the character "/" used in the disclosure generally represents "or" between the associated objects.

Other embodiments of the invention will be apparent to those skilled in the art from consideration of the specification and practice of the disclosures herein. This application is intended to cover any variations, uses, or adaptations of the disclosure following the general principles thereof and including such departures from the present disclosure as come within known or customary practice in the art. It is intended that the specification and examples be considered as exemplary only, with a true scope of the invention being indicated by the following claims.

It will be appreciated that the inventive concept is not limited to the exact construction that has been described above and illustrated in the accompanying drawings, and that various modifications and changes can be made without departing from the scope thereof. It is intended that the scope of the invention only be limited by the appended claims.

## Claims

1. A communication method for a wearable device, **characterized in** comprising:
acquiring (401) a motion state parameter of a wearable device;
detecting (402), at a current time, whether a temperature of a location where the wearable device contacts with a user's skin is higher than a temperature of another location of the wearable device; and
if the temperature of the location where the wearable device contacts with the user's skin is not higher than the temperature of the other location of the wearable device, determining (403), whether the wearable device is in a fallen state, in which the wearable device is no longer being worn because it has fallen, the determination being based on the motion state parameter within a predefined time period before the current time; and
if the wearable device is in the fallen state, sending (408) information about falling to a terminal to instruct the terminal to send out an alerting signal indicating that the wearable device has fallen,
wherein the motion state parameter comprises speed and acceleration, and the step of determining whether the wearable device is in the fallen state based on the motion state parameter comprises:
detecting (4031) whether the motion state parameter is a fallen state parameter, which indicates that the acceleration is the gravity acceleration and the speed is not 0 during a first time period while the acceleration and the speed are both 0 during a second time period, wherein the first time period and the second time period are two consecutive time periods.

2. The method of claim 1, wherein the step of determining whether the wearable device is in the fallen state based on the motion state parameter comprises:
when the motion state parameter is the fallen state parameter, determining (4032) that the wearable device is in the fallen state; and
when the motion state parameter is not the fallen state parameter, determining (4033) that the wearable device is not in the fallen state.

3. The method of claim 1 or 2, wherein the step of sending information about falling to the terminal when the wearable device is in the fallen state comprises:
when the wearable device is in the fallen state, acquiring (406) a temperature of the location where the wearable device contacts with a user's skin at current time;
determining (407) whether the temperature of the location where the wearable device contacts with the user's skin at the current time is lower than the temperature of the location where the wearable device contacts with the user's skin at a previous time, wherein the difference between the current time and the previous time is t seconds, t>0; and
when the temperature at the current time is lower than the temperature at the previous time, sending (408) the information about falling to the terminal.

4. The method of any of claims 1 to 3, wherein the information about falling comprises at least one of the location of the wearable device and fallen time of the wearable device, and the step of sending the information about falling to the terminal comprises:
determining (404) the location of the wearable device; and/or
setting (405) the time when the speed and the acceleration of the wearable device both become 0 as the fallen time of the wearable device.

5. The method of any of claims 1 to 3, further comprising:
sending out (409) a warning signal.

6. The method of claim 5, wherein the warning signal comprises at least one of a sound signal, a vibration signal and a light signal.

7. A communication apparatus for wearable device, **characterized in** comprising:
an acquisition module (501) configured to acquire a motion state parameter of a wearable device, the motion state parameter comprising speed and acceleration;
a determination module (502) configured to:
detect, at a current time, whether a temperature of a location where the wearable device contacts with a user's skin is higher than a temperature of another location of the wearable device; and
if the temperature of the location where the wearable device contacts with the user's skin is not higher than the temperature of the other location of the wearable device determine whether the wearable device is in a fallen state, in which the wearable device is no longer being worn because it has fallen, the determination being based on the motion state parameter within a predefined time period before the current time; and
if the wearable device is in the fallen state, a sending module (503) is configured to send information about falling to a terminal to instruct the terminal to send out an alerting signal indicating that the wearable device has fallen,
wherein the determination module (502) is configured to detect whether the motion state parameter is a fallen state parameter, which indicates that the acceleration is the gravity acceleration and the speed is not 0 during a first time period while the acceleration and the speed are both 0 during a second time period, wherein the first time period and the second time period are two consecutive time periods.

8. The apparatus of claim 7, wherein the determination module (502) is configured to:
when the motion state parameter is the fallen state parameter, determine that the wearable device is in the fallen state; and
when the motion state parameter is not the fallen state parameter, determine that the wearable device is not in the fallen state.

9. The apparatus of claim 7 or 8, wherein the sending module (503) is configured to:
when the wearable device is in the fallen state, acquire a temperature of the location where the wearable device contacts with a user's skin at current time;
determine whether the temperature of the location where the wearable device contacts with the user's skin at the current time is lower than the temperature of the location where the wearable device contacts with the user's skin at a previous time, wherein the difference between the current time and the previous time is t seconds, t>0; and
when the temperature at the current time is lower than the temperature at the previous time, send the information about falling to the terminal.

10. The apparatus of any of claims 7 to 9, wherein the information about falling comprises at least one of the location of the wearable device and fallen time of the wearable device and the sending module (503) is configured to:
determine the location of the wearable device; and/or
set the time when the speed and the acceleration of the wearable device both become 0 as the fallen time of the wearable device.

11. The apparatus of any of claims 7 to 10, further comprising a warning module (504) configured to send out a warning signal.

12. The apparatus of claim 11, wherein the warning signal comprises at least one of sound signal, vibration signal and light signal.

## Patentansprüche

1. Kommunikationsverfahren für ein tragbares Gerät, **dadurch gekennzeichnet, dass** es Folgendes umfasst:
Erfassen (401) eines Bewegungszustandsparameters eines tragbaren Geräts;
Erkennen (402) zu einem aktuellen Zeitpunkt, ob eine Temperatur an einer Stelle, an der das tragbare Gerät mit der Haut eines Benutzers in Kontakt ist, höher ist als die Temperatur an einer anderen Stelle des tragbaren Geräts; und
Feststellen (403), wenn die Temperatur an der Stelle, an der das tragbare Gerät mit der Haut des Benutzers in Kontakt ist, nicht höher ist als die Temperatur an der anderen Stelle des tragbaren Geräts, ob das tragbare Gerät in einem heruntergefallenen Zustand ist, in dem das tragbare Gerät nicht mehr getragen wird, weil es heruntergefallen ist, wobei die Feststellung auf dem Bewegungszustandsparameter innerhalb eines vordefinierten Zeitraums vor dem aktuellen Zeitpunkt basiert; und
Senden (408), wenn das tragbare Gerät im heruntergefallenen Zustand ist, von Informationen über das Herunterfallen zu einem Endgerät, um das Endgerät anzuweisen, ein Warnsignal auszusenden, das anzeigt, dass das tragbare Gerät heruntergefallen ist,
wobei der Bewegungszustandsparameter Geschwindigkeit und Beschleunigung umfasst und der Schritt des Feststellens auf der Basis des Bewegungszustandsparameters, ob das tragbare Gerät im heruntergefallenen Zustand ist, Folgendes beinhaltet:
Erkennen (4031), ob der Bewegungszustandsparameter ein Herunterfallzustandsparameter ist, der anzeigt, dass während eines ersten Zeitraums die Beschleunigung die Schwerkraftbeschleunigung ist und die Geschwindigkeit nicht 0 ist, während während eines zweiten Zeitraums Beschleunigung und Geschwindigkeit beide 0 sind, wobei der erste Zeitraum und der zweite Zeitraum zwei aufeinanderfolgende Zeiträume sind.

2. Verfahren nach Anspruch 1, wobei der Schritt des Feststellens auf der Basis des Bewegungszustandsparameters, ob das tragbare Gerät im heruntergefallenen Zustand ist, Folgendes beinhaltet:
Feststellen (4032), wenn der Bewegungszustandsparameter der Herunterfallzustandsparameter ist, dass das tragbare Gerät im heruntergefallenen Zustand ist; und
Feststellen (4033), wenn der Bewegungszustandsparameter nicht der Herunterfallzustandsparameter ist, dass das tragbare Gerät nicht im heruntergefallenen Zustand ist.

3. Verfahren nach Anspruch 1 oder 2, wobei der Schritt des Sendens von Informationen über das Herunterfallen zu dem Endgerät, wenn das tragbare Gerät im heruntergefallenen Zustand ist, Folgendes beinhaltet:
Erfassen (406), wenn das tragbare Gerät im heruntergefallenen Zustand ist, einer Temperatur an der Stelle, an der das tragbare Gerät zum aktuellen Zeitpunkt mit der Haut eines Benutzers in Kontakt ist;
Feststellen (407), ob die Temperatur an der Stelle, an der das tragbare Gerät zum aktuellen Zeitpunkt mit der Haut des Benutzers in Kontakt ist, niedriger ist als die Temperatur an der Stelle, an der das tragbare Gerät zu einem vorherigen Zeitpunkt mit der Haut des Benutzers in Kontakt ist, wobei die Differenz zwischen dem aktuellen Zeitpunkt und dem vorherigen Zeitpunkt t Sekunden, t>0, beträgt; und
Senden (408), wenn die Temperatur zum aktuellen Zeitpunkt niedriger ist als die Temperatur zu dem vorherigen Zeitpunkt, der Informationen über das Herunterfallen zu dem Endgerät.

4. Verfahren nach einem der Ansprüche 1 bis 3, wobei die Information über das Herunterfallen den Ort des tragbaren Geräts und/oder den Zeitpunkt des Herunterfallens des tragbaren Geräts umfasst und der Schritt des Sendens der Informationen über das Herunterfallen zu dem Endgerät Folgendes beinhaltet:
Bestimmen (404) des Orts des tragbaren Geräts; und/oder
Festlegen (405) des Zeitpunkts, an dem Geschwindigkeit und Beschleunigung des tragbaren Geräts beide 0 werden, als den Zeitpunkt des Herunterfallens des tragbaren Geräts.

5. Verfahren nach einem der Ansprüche 1 bis 3, das ferner das Aussenden (409) eines Warnsignals beinhaltet.

6. Verfahren nach Anspruch 5, wobei das Warnsignal mindestens eines aus einem Tonsignal, einem Vibrationssignal und einem Lichtsignal umfasst.

7. Kommunikationsvorrichtung für ein tragbares Gerät, **dadurch gekennzeichnet, dass** sie Folgendes umfasst:
ein Erfassungsmodul (501), konfiguriert zum Erfassen eines Bewegungszustandsparameters eines tragbaren Geräts, wobei der Bewegungszustandsparameter Geschwindigkeit und Beschleunigung umfasst;
ein Bestimmungsmodul (502), konfiguriert zum:
Erkennen, zu einem aktuellen Zeitpunkt, ob eine Temperatur an einer Stelle, an der das tragbare Gerät mit der Haut eines Benutzers in Kontakt ist, höher als die Temperatur an einer anderen Stelle des tragbaren Geräts; und
Feststellen, wenn die Temperatur an der Stelle, an der das tragbare Gerät mit der Haut des Benutzers in Kontakt ist, nicht höher ist als die Temperatur an der anderen Stelle des tragbaren Geräts, ob das tragbare Gerät in einem heruntergefallenen Zustand ist, in dem das tragbare Gerät nicht mehr getragen wird, weil es heruntergefallen ist, wobei die Feststellung auf dem Bewegungszustandsparameter innerhalb eines vordefinierten Zeitraums vor dem aktuellen Zeitpunkt basiert; und
wenn das tragbare Gerät im heruntergefallenen Zustand ist, ein Sendemodul (503) so konfiguriert ist, dass es Informationen über das Herunterfallen zu einem Endgerät sendet, um das Endgerät anzuweisen, ein Alarmsignal auszusenden, das anzeigt, dass das tragbare Gerät heruntergefallen ist,
wobei das Feststellungsmodul (502) so konfiguriert ist, dass es erkennt, ob der Bewegungszustandsparameter ein Herunterfallzustandsparameter ist, der anzeigt, dass während eines ersten Zeitraums die Beschleunigung die Schwerkraftbeschleunigung ist und die Geschwindigkeit nicht 0 ist, während während eines zweiten Zeitraums die Beschleunigung und die Geschwindigkeit beide 0 sind, wobei der erste Zeitraum und der zweite Zeitraum zwei aufeinanderfolgende Zeiträume sind.

8. Vorrichtung nach Anspruch 7, wobei das Feststellungsmodul (502) konfiguriert ist zum:
Feststellen, wenn der Bewegungszustandsparameter der Herunterfallzustandsparameter ist, dass das tragbare Gerät im heruntergefallenen Zustand ist; und
Feststellen, wenn der Bewegungszustandsparameter nicht der Herunterfallzustandsparameter ist, dass das tragbare Vorrichtung nicht im heruntergefallenen Zustand ist.

9. Vorrichtung nach Anspruch 7 oder 8, wobei das Sendemodul (503) konfiguriert ist zum:
Erfassen, wenn das tragbare Gerät im heruntergefallenen Zustand ist, einer Temperatur der Stelle, an der das tragbare Gerät zum aktuellen Zeitpunkt mit der Haut eines Benutzers in Kontakt ist;
Feststellen, ob die Temperatur an der Stelle, an der das tragbare Gerät mit der Haut des Benutzers zum aktuellen Zeitpunkt in Kontakt ist, niedriger ist als die Temperatur an der Stelle, an der das tragbare Gerät mit der Haut des Benutzers zu einem vorherigen Zeitpunkt in Kontakt ist, wobei die Differenz zwischen der aktuellen Zeit und der vorherigen Zeit t Sekunden, t>0, ist; und
Senden, wenn die Temperatur zum aktuellen Zeitpunkt niedriger ist als die Temperatur zum vorherigen Zeitpunkt, der Informationen über das Herunterfallen zu dem Endgerät.

10. Vorrichtung nach einem der Ansprüche 7 bis 9, wobei die Informationen über das Herunterfallen den Ort des tragbaren Geräts und/oder den Zeitpunkt des Herunterfallens des tragbaren Geräts umfasst und das Sendemodul (503) konfiguriert ist zum:
Bestimmen des Orts des tragbaren Geräts; und/oder
Festlegen des Zeitpunkts, an dem Geschwindigkeit und Beschleunigung des tragbaren Geräts beide 0 werden, als den Zeitpunkt des Herunterfallens des tragbaren Geräts.

11. Vorrichtung nach einem der Ansprüche 7 bis 10, die ferner ein Warnmodul (504) umfasst, konfiguriert zum Aussenden eines Warnsignals.

12. Vorrichtung nach Anspruch 11, wobei das Warnsignal mindestens eines aus Tonsignal, Vibrationssignal und Lichtsignal umfasst.

## Revendications

1. Procédé de communication pour un dispositif vestimentaire, **caractérisé en ce qu'**il comprend :
l'acquisition (401) d'un paramètre d'état de mouvement d'un dispositif vestimentaire ;
la détection (402) à un temps actuel, qu'une température d'un emplacement où le dispositif vestimentaire fait contact avec la peau d'un utilisateur est supérieure ou non à une température d'un autre emplacement du dispositif vestimentaire ; et
si la température de l'emplacement où le dispositif vestimentaire fait contact avec la peau de l'utilisateur n'est pas supérieure à la température de l'autre emplacement du dispositif vestimentaire, la détermination (403), que le dispositif vestimentaire a ou non chuté, le dispositif vestimentaire n'étant plus porté du fait de sa chute, la détermination étant basée sur le paramètre d'état de mouvement dans une période de temps prédéfinie avant le temps actuel ; et
si le dispositif vestimentaire est dans l'état de chute, l'envoi (408) d'informations relatives à la chute à un terminal pour ordonner au terminal d'envoyer un signal d'alerte indiquant que le dispositif vestimentaire a chuté,
dans lequel le paramètre d'état de mouvement comprend la vitesse et l'accélération, et l'étape de détermination que le dispositif vestimentaire a ou non chuté en fonction du paramètre d'état de mouvement comprend :
la détection (4031) que le paramètre d'état de mouvement est un paramètre d'état de chute, lequel indique que l'accélération est l'accélération gravitaire et la vitesse n'est pas 0 durant une première période de temps tandis que l'accélération et la vitesse sont toutes les deux 0 durant une seconde période de temps, la première période de temps et la seconde période de temps étant deux périodes de temps consécutives.

2. Procédé selon la revendication 1, dans lequel l'état de détermination que le dispositif vestimentaire est ou non dans l'état de chute en fonction du paramètre d'état de mouvement comprend :
quand le paramètre d'état de mouvement est le paramètre d'état de chute, la détermination (4032) que le dispositif vestimentaire est dans l'état de chute ; et
quand le paramètre d'état de mouvement n'est pas le paramètre d'état de chute, la détermination (4033) que le dispositif vestimentaire n'est pas dans l'état de chute.

3. Procédé selon la revendication 2, dans lequel l'étape d'envoi d'informations relatives à la chute au terminal quand le dispositif vestimentaire est dans l'état de chute comprend :
quand le dispositif vestimentaire est dans l'état de chute, l'acquisition (406) d'une température de l'emplacement où le dispositif vestimentaire fait contact avec la peau d'un utilisateur à un temps actuel ;
la détermination (407) que la température de l'emplacement où le dispositif vestimentaire fait contact avec la peau de l'utilisateur au temps actuel est inférieure ou non à la température de l'emplacement où le dispositif vestimentaire fait contact avec la peau de l'utilisateur à un temps précédent, la différence entre le temps actuel et le temps précédent étant de t secondes, t > 0 ; et
quand la température au temps actuel est inférieure à la température au temps précédent, l'envoi (408) des informations relatives à la chute au terminal.

4. Procédé selon l'une quelconque des revendications 1 à 3, dans lequel les informations relatives à la chute comprennent au moins un de l'emplacement du dispositif vestimentaire et du temps de chute du dispositif vestimentaire, et l'étape d'envoi des informations relatives à la chute au terminal comprend :
la détermination (404) de l'emplacement du dispositif vestimentaire ; et/ou
l'établissement (405) du temps auquel la vitesse et l'accélération du dispositif vestimentaire deviennent toutes les deux 0 en tant que temps de chute du dispositif vestimentaire.

5. Procédé selon l'une quelconque des revendications 1 à 3, comprenant en outre :
l'envoi (409) d'un signal d'avertissement.

6. Procédé selon la revendication 5, dans lequel le signal d'avertissement comprend au moins un d'un signal sonore, d'un signal vibratoire et d'un signal lumineux.

7. Appareil de communication pour un dispositif vestimentaire, **caractérisé en ce qu'**il comprend :
un module d'acquisition (501) configuré pour acquérir un paramètre d'état de mouvement d'un dispositif vestimentaire, le paramètre d'état de mouvement comprenant la vitesse et l'accélération ;
un module de détermination (502) configuré pour:
détecter, à un temps actuel, qu'une température d'un emplacement où le dispositif vestimentaire fait contact avec la peau d'un utilisateur est supérieure ou non à une température d'un autre emplacement du dispositif vestimentaire ; et
si la température de l'emplacement où le dispositif vestimentaire fait contact avec la peau de l'utilisateur n'est pas supérieure à la température de l'autre emplacement du dispositif vestimentaire, déterminer que le dispositif vestimentaire a ou non chuté, le dispositif vestimentaire n'étant plus porté du fait de sa chute, la détermination étant basée sur le paramètre d'état de mouvement dans une période de temps prédéfinie avant le temps actuel ; et
si le dispositif vestimentaire est dans l'état de chute, un module d'envoi (503) est configuré pour envoyer des informations relatives à la chute à un terminal pour ordonner au terminal d'envoyer un signal d'alerte indiquant que le dispositif vestimentaire a chuté,
dans lequel le module de détermination (502) est configuré pour détecter que le paramètre d'état de mouvement est un paramètre d'état de chute, lequel indique que l'accélération est l'accélération gravitaire et la vitesse n'est pas 0 durant une première période de temps tandis que l'accélération et la vitesse sont toutes les deux 0 durant une seconde période de temps, la première période de temps et la seconde période de temps étant deux périodes de temps consécutives.

8. Appareil selon la revendication 7, dans lequel le module de détermination (502) est configuré pour :
quand le paramètre d'état de mouvement est le paramètre d'état de chute, déterminer que le dispositif vestimentaire est dans l'état de chute ; et
quand le paramètre d'état de mouvement n'est pas le paramètre d'état de chute, déterminer que le dispositif vestimentaire n'est pas dans l'état de chute.

9. Procédé selon la revendication 7 ou 8, dans lequel le module d'envoi (503) est configuré pour :
quand le dispositif vestimentaire est dans l'état de chute, acquérir une température de l'emplacement où le dispositif vestimentaire fait contact avec la peau d'un utilisateur à un temps actuel ;
déterminer que la température de l'emplacement où le dispositif vestimentaire fait contact avec la peau de l'utilisateur au temps actuel est inférieure ou non à la température de l'emplacement où le dispositif vestimentaire fait contact avec la peau de l'utilisateur à un temps précédent, la différence entre le temps actuel et le temps précédent étant de t secondes, t > 0 ; et
quand la température au temps actuel est inférieure à la température au temps précédent, envoyer les informations relatives à la chute au terminal.

10. Appareil selon l'une quelconque des revendications 7 à 9, dans lequel les informations relatives à la chute comprennent au moins un de l'emplacement du dispositif vestimentaire et du temps de chute du dispositif vestimentaire, et le module d'envoi (503) est configuré pour:
déterminer l'emplacement du dispositif vestimentaire ; et/ou
établir le temps auquel la vitesse et l'accélération du dispositif vestimentaire deviennent toutes les deux 0 en tant que temps de chute du dispositif vestimentaire.

11. Appareil selon l'une quelconque des revendications 7 à 10, comprenant en outre un module d'avertissement (504), configuré pour envoyer un signal d'avertissement.

12. Appareil selon la revendication 115, dans lequel le signal d'avertissement comprend au moins un d'un signal sonore, d'un signal vibratoire et d'un signal lumineux.
